# EUROPEAN PATENT SPECIFICATION

(11) **EP 1 204 422 B1**
(45) Date of publication and mention of the grant of the patent: **25.11.2009**
(21) Application number: 00954741.5
(22) Date of filing: 18.08.2000
(51) Int. Cl.: A61K 39/00, A61K 39/002, A61K 39/02, A61P 31/04, A61P 31/10, A61P 33/02

(54) **STRESS-PROTEINS FROM EXTRA-CELLULAR PATHOGENS AS VACCINES AGAINST INFECTIOUS AGENTS**
STRESS-PROTEINE EXTRAZELLULÄRER PATHOGENE ALS IMPFSTOFFE GEGEN INFEKTIÖSE ERREGER
PROTEINES DE STRESS DE PATHOGENES EXTRACELLULAIRES EN TANT QUE VACCINS CONTRE LES AGENTS INFECTIEUX

(30) Priority: 19.08.1999 GB 9919734
(43) Date of publication of application: 15.05.2002
(73) Proprietor: Immunobiology Limited, Cambridge, Cambridgeshire CB22 3AT (GB)
(72) Inventor: COLACO, Camilo, Anthony, Leo, Selwyn, Cambridge CB2 2JN (GB)
(74) Representative: Stark, Gordon Drummond
(86) International application number: PCT/GB2000/003228
(87) International publication number: WO 2001/013944

(56) References cited:
- WO-A-90/02564
- WO-A-96/40928
- WO-A2-95/24923
- FERRERO RICHARD L ET AL: "The GroES homolog of Helicobacter pylori confers protective immunity against mucosal infection in mice." PROCEEDINGS OF THE NATIONAL ACADEMY OF SCIENCES OF THE UNITED STATES, vol. 92, no. 14, 1995, pages 6499-6503, XP002160650 1995 ISSN: 0027-8424
- COLACO C.: 'Function of fever' JOURNAL OF THE ROYAL SOCIETY OF MEDICINE vol. 90, 1997, pages 587 - 588
- COLACO C.: 'BCG HspCs induce T-helper 1 responses and protect against live challenge in a murine aerosol challenge model of pulmonary tuberculosis' BIOCHEMICAL SOCIETY TRANSACTIONS vol. 32, 2004, pages 626 - 628

## Description

The present invention relates to a vaccine and a method for producing a vaccine, notably to a method for producing a vaccine composition containing stress-induced proteins from extra-cellular pathogenic organisms.

### BACKGROUND OF THE INVENTION

An important component of any human immune response is the presentation of antigens to T cells by antigen presenting cells (APCs), such as macrophages, B cells or dendritic cells. Peptide fragments of foreign antigens are presented on the surface of the macrophage in combination with major histocompatibility complex (MHC) molecules, in association with helper molecules, such as CD4 and CD8 molecules. Such antigenic peptide fragments presented in this way are recognised by the T cell receptor of T cells and the interaction of the antigenic peptide fragments with the T cell receptor results in antigen-specific T cell proliferation and secretion of lymphokines by the T cells. The nature of the antigenic peptide fragment presented by the APCs is critical in establishing immunity.

Heat shock proteins (HSPs) form a family of highly conserved proteins that are widely distributed throughout the plant and animal kingdoms. On the basis of their molecular weights, HSPs are grouped into six different families: small (hsp 20-30kDa); hsp40; hsp60; hsp70; hsp90; and hsp100. Although HSPs were originally identified in cells subjected to heat stress, they have been found to be associated with many other forms of stress such as infections, and are thus more commonly known as stress proteins (SPs). For convenience, the initials SP will be used to denote in general proteins induced from cells subjected to any form of stress and the initials HSP will be used to denote those specifically induced by heat stress.

Members of the mammalian hsp90 family include cytosolic hsp90 (hsp83) and the endoplasmic reticulum counterparts hsp90 (hsp83), hsp87, Grp94 (Erp99) and gp97, see for instance, Gething et al. (1992) Nature 355:33-45. Members of the hsp70 family include cytosolic hsp70 (p73) and hsp70 (p72), the endoplasmic reticulum counterpart BiP (Grp78), and the mitochondrial counterpart hsp70 (Grp75). Members of the mammalian hsp60 family have only been identified in the mitochondria. The latter family of HSPs is also found in procaryotes which also contain three other major families of HSPs, the GroEL, GroES, DnaJ and DnaK families. As in eucaryotes, the procaryotic HSPs are also thought to function in the folding of nascent polypeptide chains during protein synthesis.

In eucaryotic cells which have intracellular membrane organelles, one of the roles of HSPs is to chaperone antigenic peptide fragments from one cellular compartment to another and to present the peptide fragments to the MHC molecules for cell surface presentation to the immune system. In the case of diseased cells, HSPs also chaperone viral or tumour-associated peptide fragments to the cell-surface, Li and Sirivastave (1994) Behring Inst. Mitt, 94: 37-47 and Suzue et al. (1997) Proc.Natl.Acad.Sci. USA 94: 13146-51. The chaperone function is accomplished through the formation of complexes between HSPs and the antigenic peptide fragments and between HSPs and viral or tumour-associated peptide fragments in an ATP-dependent reaction. HSPs form complexes or bind with a wide spectrum of peptide fragments in an ATP dependent manner. The bound peptides appear to be a random mix of peptide fragments. The mixtures and exact natures of the peptide fragments have not been determined. The association of HSPs with various peptide fragments has been observed in normal tissues as well and is not a tumour-specific phenomenon, see Srivastava (1994) Experimentia 50: 1054-60.

In a therapeutic context, it has been proposed to use mammalian HSPs as vaccines. WO 97/10000 and WO 97/10001 disclose that a mixture of HSPs isolated from cancer cells or virally infected cells are capable eliciting protective immunity or cytotoxic T lymphocytes to the cognate tumour or viral antigen. However, in contrast, HSPs isolated from normal cells are unable to elicit such immunity. It is now thought that HSPs are not immunogenic *per se*, but are able to elicit immunity because of their association with tumour or virus specific antigenic peptide fragments that are generated during antigen processing. Specifically, the peptide fragments associated with the HSPs are immunogenic and are presented to the T cells. HSPs stripped of associated peptide fragments lose their immunogenicity, see Udono, H. and Srivastava, P. K., Journal of Experimental Medicine, 178, page 1391 ff, 1993. To date, the nature of these peptide fragments has not been determined.

It is currently believed that the antigenicity of SPs results not from the SP per se, but from the complex of peptide fragments associated with the SP. This conclusion is based on a number of characteristics of the complexes. There are no differences in the structure of SPs derived from normal and tumour cells. Certain complexes lose their immunogenicity upon treatment with ATP, Udono et al. (1993) J.Exp.Med. 178: 1391-96. Such loss of immunogenicity is due to dissociation of the complex into its SP and peptide fragment components. The immunogenicity of SP preparations depends upon the presence of phagocytic cells, such as macrophages and other APCs. It is now thought that SPs are taken up by macrophages. Those peptide fragments associated with the SPs are then presented by MHC class I molecules of the macrophage. In this way, a T cell response is initiated.

The use of mammalian HSP-complexes from infected cells as vaccines against intracellular pathogens has been disclosed in WO 95/24923. HSPs isolated from virally infected cells have been suggested as a source of antigenic peptides, which could then be presented to T cells. This necessitates the production and purification of HSPs from such cells. The use of HSP proteins as vaccine components has further been disclosed in WO 97/10000, WO 97/10001 and WO 97/100002. These disclose that a mixture of HSPs isolated from cancer cells or virally infected cells are capable of eliciting protective immunity or cytotoxic T lymphocytes to the cognate tumour or viral antigen. Furthermore WO 98/34641 discloses that surprisingly low amounts of HSPs are required to immunise animals against tumour or viral antigens.

All these HSP vaccine approaches utilise mammalian HSPs from the species to be immunised for the vaccination of the desired animal species.

HSPs from extra-cellular pathogens themselves have also been utilised to immunise mammalian species as antigens *per se* but not as carriers of antigenic peptide fragments except as conjugates or hybrid fusion proteins. Thus WO 95/14093 discloses that the use of Helicobacter pylori HspA and B as immunogens elicits a good antibody response against these proteins and that this response is effective against the organism. Similarly, WO 96/40928 discloses that the use of HSP 70 and 72 from Streptococcus elicits a good antibody response against these proteins and that this response is effective against the organism. Furthermore WO 90/02564 discloses that the use of Trypanosomal, Mycoplasmal or Mycobacterial HSPs, and especially HSP70, as immunogens elicits a good antibody response against these proteins and that this should be effective against the respective organisms. Alternatively US 5830475 uses proteins expressed as fusions of the M.Bovis HSP genes as antigens and US 5736164 uses the T-cell epitope of hsp65 conjugated to poorly immunogenic antigens.

However, endogenous SP-complexes from extra-cellular procaryotic and protozoan pathogenic species, and more especially HSP-complexes from these organisms treated by heat shock or other stresses, have not been used as vaccines to immunise animals, notably vertebrates such as mammals, birds or fish against these infectious disease pathogens.

### SUMMARY OF THE INVENTION:

Therefore, from a first aspect, the present invention provides a method for producing a vaccine containing an immunogenic determinant, comprising the steps of:
a) exposing procaryotic, protozoa or fungal pathogenic organisms to stress inducing stimuli, such as heat, which would induce the production of SP/antigenic peptide fragment complexes;
b) extracting the endogenous SP/antigenic peptide fragment complexes, from the treated organisms; and
c) using the extracted stress protein/antigenic peptide fragment complexes as the immunogenic determinant in the preparation of the vaccine composition.

It is surprising that the treatment of procaryotic, protozoa or fungal pathogen organisms with stress-inducing stimuli produces SP complexes which are more immunogenic than the SPs themselves or SPs derived from uninduced organisms. A particular property of the vaccines of the invention is the exceptionally high neutralising antibody titres and the long-term memory obtained compared to that induced by immunisation by the SPs themselves.

The term vaccine is used herein to denote to any composition which stimulates the immune system such that it can better respond to subsequent infections. It will be appreciated that a vaccine usually contains an immunogenic determinant (the stress induced SP complexes) and an adjuvant, which non-specifically enhances the response to that determinant.

The term extra-cellular pathogenic organism is used herein to denote any extra-cellular pathogen that causes a disease in a vertebrate, including bacterial, procaryotic, protozoal and fungal species. Specific examples of extra-cellular pathogens to which the method of the invention may be applied include bacteria such as Mycobacteria sp., notably M Bovis and M Tuberculosis, Helicobacter sp., Streptococcus sp., Trypanosoma sp., Mycoplasma sp.; and procaryotic pathogens such as Escherichia sp, notably E coli, and Salmonella sp., notably S typhimurium.

The extra-cellular pathogen may be one in which the application of an external stress induces the synthesis of stress proteins. However, it is within the scope of the present invention to use pathogenic organisms, for example bacteria, which have been modified, for example genetically engineered, to produce an organism in which the induction or enhancement of the induction of the sythesis of stress proteins occurs constitutively without the need to apply external stresses.

Also described herein is a method for eliciting an immune response from an animal to infection by an intra-cellular pathogenic organism which comprises administering a vaccine containing an immunogenic determinant, characterised in that the immunogenic determinant is an SP/antigenic peptide fragment complex produced in situ from the intra-cellular pathogen whose synthesis is induced by external stimuli or by genetic modification of the pathogen so as to render its synthesis constitutive.

Also described herein is an intra-cellular pathogenic organism which has had its genetic structure modified so as to remove or inhibit that portion of its genetic structure' which restricts or inhibits the synthesis of stress proteins by that organism.

The terms stress proteins and heat shock protein, as used herein, are standard in the art and include those proteins that comprise the GroEL, GroES and DnaK and DnaJ families of bacterial HSPs and related families in other extra-cellular pathogens. These families are named on the basis of the size of the peptides which they encode. The families are highly conserved between species. In addition, many bacteria also express homologues of eucaryotic proteins. Preferably the vaccine contains a plurality of SP/antigenic peptide fragment complexes derived from the stressed pathogen. We particularly prefer that the GroEL, GroES, DnaK and DnaJ families of proteins are used as immunogenic determinants in the present invention, with DnaJ and GroEL most preferred.

The stress stimuli to which the extra-cellular pathogen is exposed may be applied by any suitable in vitro technique used in the immunobiology art, for example cultivation under limited nutrient levels, or osmotic shock of a pathogen once it has been cultivated to stationary growth by the addition of high concentrations of an electrolyte such as NaCl to the cultivation medium. We prefer to apply the stress by a heat treatment of the pathogen at a temperature 5-8°C above the normal growth temperature of the organism. Typically, the pathogen will be cultivated under conventional growth conditions to the stationary state. Samples of the active pathogen culture can then be taken and cultivated again but the temperature of cultivation is increased during the second cultivation stage to the elevated temperature required to induce production of the SPs. Without being constrained by theory, it is thought that the treatment of the pathogen operates either to induce specifically those HSPs most able to interact with antigenic peptides, or to induce those HSPs which are most easily phagocytosed by APCs, or both. The optimum conditions for inducing the SPs can readily be determined by simple trial and error and the effect of a change of stimuli assessed using conventional techniques, such as in vivo testing on animals or by other techniques, for example those described in 'Current Protocols in Immunology', Wiley Interscience, 1997.

The extraction and purification of protein materials induced from the extra-cellular pathogens by the applied stress, notably the SP/antigenic peptide fragment complexes, from the remaining extra-cellular pathogen material can be achieved using any suitable technique. For example, the treated organism can be disrupted by homogenisation or ultrasonic fragmentation, followed by centrifugation to obtain a crude SP preparation in the supernatant. The crude endogenous SP preparations may be used directly as the vaccine of the invention. Optionally, the SP preparations may be purified further by the use of ADP binding columns or other suitable methods readily available to the person skilled in the art, see for example those described in WO 97/10000 and WO 97/10001.

It will be appreciated that specific immunogenic SP/antigenic peptide fragment complexes can be isolated from the mixture of complexes produced from the stressing of the extra-cellular pathogenic organisms to produce a vaccine with is pathogen specific. However, this well usually not be required and the mixture of complexes can be used to induce broad spectrum immunisation. If desired, the specific antigenic peptide fragments can be recovered from the complex, for example by treatment with ATP using conventional techniques.

The SP/antigenic peptide fragment complex of the vaccine of the present invention may be delivered in combination with an adjuvant and in an aqueous carrier. Suitable adjuvants are readily apparent to the person skilled in the art, such as Freund's complete adjuvant, Freund's incomplete adjuvant, Quil A, Detox, ISCOMs or squalene. However, the vaccine compositions of the present invention may also be effective without an adjuvant.

The invention also provides a method for treating an animal with a vaccine of the invention by administering a pharmaceutically acceptable quantity of the vaccine of the invention, optionally in combination with an adjuvant, sufficient to elicit an immune response in the animal. The animal is typically a human. However, the invention can also be applied to the treatment of other mammals such as horses, cattle, goats, sheep or swine, and to the treatment of birds, notably poultry such as chicken or turkeys.

Vaccine compositions may be administered by any suitable means, such as orally, by inhalation, transdermally or by injection and in any suitable carrier medium. However, it is preferred to administer the vaccine as an aqueous composition by injection using any suitable needle or needle-less technique.

Vaccines may contain any suitable concentration of the SP/antigenic peptide fragment complex. We prefer that the SP complex is administered in the range of 10-600 µg, preferably 10-100 µg, most preferably 25 µg, per Kg of body weight of the animal being treated. It will be appreciated that the vaccine may be applied as an initial treatment followed by one or more subsequent treatments at the same or a different dosage rate at an interval of from 1 to 26 weeks between each treatment to provide prolonged immunisation against the pathogen.

The following examples are provided to illustrate but not limit the invention.

### Example 1: Preparation of heat-induced HSPs:

Cells of the extra-cellular pathogen *Mycobacterium Bovis* (BCG) were grown to stationary phase using a conventional cultivation medium at 37°C and heat-shocked at 42°C for 0.5hr or at 39°C for 5hr and cultured overnight to induce the formation of a product containing heat shock protein and antigenic peptide fragments. The cells of the pathogen are then washed in phosphate buffered saline (PBS) and re-suspended in homogenisation buffer, notably a hypotonic buffer such as 10 mM phosphate pH 7.4 with 2mM MgCl₂. The cells are then disrupted using any suitable technique: for example using a cell homogeniser such as a French press, Ultraturrax or Waring blender; by lysis using detergents such as Tween or Triton; complement lysis at 37°C; or by repeated freeze-thaw cycles, e.g. in liquid nitrogen. The cell lysate is then treated by centrifugation, typically at 3-5000g for 5 minutes, to remove the nuclear and cell debris, followed by a high speed centrifugation step, typically 100,000g for 15-30 minutes.

The supernatant thus obtained contains, inter alia, the heat shock protein and the antigenic peptide fragments induced by the heat shock treatment of the pathogen cells. This can be used directly to form the active component of the vaccine composition of the invention. The supernatant may be concentrated using any suitable technique to produce the vaccine composition. Alternatively, the supernatant may be further processed by ammonium sulphate precipitation which uses a 20-70% ammonium sulphate cut. Specifically, 20% (w/w) ammonium sulphate is added at 4°C, the precipitate is discarded, followed by the addition of more ammonium sulphate to bring the concentration to 70%w/w. The protein precipitate is harvested by centrifugation and then dialysed into an appropriate physiological, injectable buffer, such as saline, to remove the ammonium sulphate before use. It will be appreciated that the HSPs isolated in this way are not purified to homogeneity, but are nevertheless suitable for use as a vaccine component.

If a more purified HSP preparation is required, then the HSPs may be purified from the supernatant by affinity chromatography on matrices carrying adenosine diphosphate, such as ADP-agarose or ADP-sepharose, for example as described in WO 97/10000, WO 97/10001 and WO 97/10002.

In order to determine the immunogenicity of the stress protein/antigenic peptide fragment complexes produced as described above, T cell proliferation assays may be used. Suitable assays include the mixed-lymphocyte reaction (MLR), assayed by tritiated thymidine uptake; and cytotoxicity assays to determine the release of ⁵¹Cr from target cells. Both of these assays are standard in the art, see 'Current Protocols in Immunology', Wiley Interscience, 1997. Alternatively, antibody production may be examined, using standard immunoassays or plaque-lysis assays, or assessed by intrauterine protection of a foetus, see 'Current Protocols in Immunology'.

### Example 2: Immunisation with induced HSPs; immunity in vaccine recipient.

Vaccine compositions containing HSP complexes were prepared as described in Example 1 above and mice and rabbits vaccinated by injection of 1-10 micrograms of the stress protein complex in phosphate buffered saline. This initial immunisation was boosted with identical vaccine dosages a month after the primary injection. Induction of immunity to pathogen was assayed by Western blot analysis using total *M.bovis* proteins. Antibody titres of 1:1-10,000 were routinely obtained and cytotoxic T-cell activity directed against pathogen infected cells could also be detected in the immunised mice. Challenge of the rabbits with fixed *M.bovis* at 6, 12 and 18 months periods after the initial immunisations resulted in the production of good antibody responses with titres of 1:1-10 000 indicating good memory responses in the immunised animals.

### Example 3: Comparison of associated peptides in constitutive and induced HSP complexes

*Mycobacterium Tuberculosis* was grown to saturation for 3 days at 37°C in Sauton's medium. 4ml aliquots of the stationary cultures were used to inoculate 500ml of Sauton's medium in a 2 litre conical flask and the cultures grown overnight at 30°C. The log phase cultures were then raised to 40°C and grown for a further 4hrs before the bacteria were harvested by centrifugation at 10,000 rpm for 10 minutes. Non-induced (constitutive) HSPs were isolated by centrifugation from the initial cell cultures at 37°C.

Cell pellets from the centrifuged samples were re-suspended in lysis solution containing 0.5% Tween and HSPs prepared from induced and non-induced cells using ammonium sulphate precipitation as in Example 1 above. The purified HSPs were re-suspended in 10% acetic acid and boiled for 15mins to elute HSP-associated peptides. The denatured HSPs were pelleted in a Beckman airfuge for 30mins in a cold room and the peptide-containing supernatants harvested by freeze-drying and analysed by capillary zone electrophoresis using a Beckman CZE system. The CZE profiles of the peptides eluted from constitutive and heat-induced *M.Tuberculosis* HSPs were significantly different indicating that they carried distinct families of associated peptides. Immunisation of mice with the heat-induced HSPs gave significantly better immunity, as assessed by lung colony counts, to live challenge than immunisation with constitutive HSPs.

### Example 4: Use of induced procaryotic HSP complexes as vaccines.

*E.Coli* (NCIMB strain 9484) and *Salmonella typhimurium* (strain 1344) were grown overnight at 37°C in LB medium. 4ml aliquots of the stationary cultures were used to inoculate 200ml of LB medium in a 2 litre conical flask and the cultures grown for 3hrs at 30°C. The log phase cultures were then raised to 40°C and grown for a further 3hrs before the bacteria were harvested by centrifugation at 10,000 rpm for 10 minutes to give pellets of heat shock proteins. Similarly, pellets of non-heat shocked (constitutive) proteins were prepared from the initial cell cultures at 37°C.

Cell pellets were re-suspended in lysis solution of 0.5% Tween in 10mM Tris-HCl, pH8 and HSPs prepared from induced and non-induced cells using ammonium sulphate precipitation as in Example 1 above. Immunisation of rabbits with the intact HSP-peptide complexes from non-induced and heat-induced bacteria showed a 10-100 fold antibody titres in the animals immunised with HSPs from heat-induced bacteria as assessed in dot-blot assays using the isolated HSPs. In control experiments, animals were immunised with the reconstituted mixture of the denatured HSPs and the peptides eluted from them prepared as described in Example 3 above for CZE analysis. Surprisingly, no difference in the antibody titres was seen between the reconstituted mixes prepared from constitutive or heat-induced HSPs-complexes indicating that the enhanced immune responses seen with native heat-induced HSP-peptide complexes was due to the in situ formed complexes and not simply due to an adjuvant property of the HSP component itself.

## Claims

1. A method for producing a vaccine composition comprising an immunogenic determinant, comprising the steps of:
- exposing procaryotic, protozoan or fungal pathogenic organisms to stress-inducing stimuli sufficient to induce the production of endogenous stress protein/antigenic peptide fragment complexes;
- extracting the endogenous stress protein/antigenic peptide fragment complexes from the treated organisms; and
- using the extracted stress protein/antigenic peptide fragment complexes as the immunogenic determinant in the preparation of the vaccine composition.

2. A method as claimed in claim 1 **characterised in that** the stress-inducing stimulus is heat.

3. A method as claimed in claim 2, **characterised in that** the pathogenic organism is heated to from 5 to 8°C above the normal temperature for cultivation of the organism.

4. A method as claimed in any one of the preceding claims, **characterised in that** the pathogenic organism is a bacterial pathogenic organism.

5. A method as claimed in any one of the preceding claims, **characterised in that** the pathogenic organism has been genetically modified to induce or enhance the induction of the synthesis of stress proteins.

6. Use of a vaccine composition produced by the method of any one of claims 1 to 5 in the preparation of vaccine composition for eliciting an immune response in an animal against the pathogenic organism from which the stress protein/antigenic peptide fragment complex, which is the immunogenic determinant in the vaccine composition, is derived.

## Patentansprüche

1. Ein Verfahren zum Produzieren einer Impfstoffzusammensetzung, die eine immunogene Determinante beinhaltet, wobei das Verfahren die folgenden Schritte beinhaltet:
- Aussetzen von prokaryotischen, Protozoen oder Pilz betreffenden pathogenen Organismen gegenüber stressinduzierenden Stimuli, die ausreichend sind, um die Produktion von endogenen Stressprotein-/Antigenpeptidfragmentkomplexen zu induzieren;
- Extrahieren der endogenen Stressprotein-/Antigenpeptidfragmentkomplexe aus den behandelten Organismen; und
- Verwenden der extrahierten Stressprotein-/Antigenpeptidfragmentkomplexe als die immunogene Determinante bei der Zubereitung der Impfstoffzusammensetzung.

2. Verfahren gemäß Anspruch 1, **dadurch gekennzeichnet, dass** der stressinduzierende Stimulus Hitze ist.

3. Verfahren gemäß Anspruch 2, **dadurch gekennzeichnet, dass** der pathogene Organismus auf 5 bis 8 °C über der Normaltemperatur zur Kultivierung des Organismus erhitzt wird.

4. Verfahren gemäß einem der vorhergehenden Ansprüche, **dadurch gekennzeichnet, dass** der pathogene Organismus ein bakterieller pathogener Organismus ist.

5. Verfahren gemäß einem der vorhergehenden Ansprüche, **dadurch gekennzeichnet, dass** der pathogene Organismus genetisch modifiziert worden ist, um die Induktion der Synthese von Stressproteinen zu induzieren oder zu erhöhen.

6. Verwendung einer durch das Verfahren gemäß einem der Ansprüche 1 bis 5 produzierten Impfstoffzusammensetzung bei der Zubereitung der Impfstoffzusammensetzung zum Auslösen einer Immunreaktion bei einem Lebewesen gegen den pathogenen Organismus, aus dem der Stressprotein-/Antigenpeptidfragmentkomplex, der die immunogene Determinante bei der Impfstoffzusammensetzung ist, erlangt wird.

## Revendications

1. Une méthode pour produire une composition de vaccin comprenant un déterminant immunogène, comprenant les étapes consistant à :
- exposer des organismes pathogènes procaryotes, protozoaires ou fongiques à des stimuli inducteurs de stress suffisants pour induire la production de complexes endogènes protéines de stress / fragments de peptides antigènes ;
- extraire les complexes endogènes protéines de stress / fragments de peptides antigènes des organismes traités ; et
- utiliser les complexes protéines de stress / fragments de peptides antigènes extraits comme déterminant immunogène dans la préparation de la composition de vaccin.

2. Une méthode telle que revendiquée dans la revendication 1 **caractérisée en ce que** le stimulus inducteur de stress est de la chaleur.

3. Une méthode telle que revendiquée dans la revendication 2, **caractérisée en ce que** l'organisme pathogène est chauffé à une température allant de 5 à 8 °C au-dessus de la température de culture normale de l'organisme.

4. Une méthode telle que revendiquée dans n'importe laquelle des revendications précédentes, **caractérisée en ce que** l'organisme pathogène est un organisme pathogène bactérien.

5. Une méthode telle que revendiquée dans n'importe laquelle des revendications précédentes, **caractérisée en ce que** l'organisme pathogène a été génétiquement modifié pour induire ou renforcer l'induction de la synthèse de protéines de stress.

6. Utilisation d'une composition de vaccin produite par la méthode de n'importe laquelle des revendications 1 à 5 dans la préparation de composition de vaccin pour éliciter une réponse immunitaire chez un animal contre l'organisme pathogène duquel le complexe protéines de stress / fragments de peptides antigènes, lequel est le déterminant immunogène dans la composition de vaccin, est dérivé.
